# EUROPEAN PATENT APPLICATION

(11) **EP 1 332 723 A1**
(43) Date of publication of application: **06.08.2003**
(21) Application number: 02258323.1
(22) Date of filing: 03.12.2002
(51) Int. Cl.: A61B 18/14

(54) **Diathermy forceps connection means**

(30) Priority: 03.12.2001 GB 0128865
(71) Applicant: Trust Sterile Services Limited, Bellshill, Lanarkshire ML4 3NJ (GB)
(72) Inventor: Heneaghan, John G., Whitecraigs, Glasgow G46 6SF (GB)
(74) Representative: McKechnie, Neil Henry

(57) **Abstract**

A diathermy forceps power supply connection means (5) is described that is suitable for use with either a bipolar or a mono-polar power supply. Employing such a connection means permits a user to employ the same set of diathermy forceps (1) and electrical supply cables (9) with either the bipolar or mono-polar power supply. Therefore, the user can quickly and efficiently transfer the operation of the diathermy forceps (1) so as to operate in either a bipolar of mono-polar mode.

## Description

The present invention relates to the field of connection means for diathermy forceps. In particular, it relates to a diathermy forceps connection means that allows an associated power supply to be either bipolar or mono-polar.

The Prior Art teaches of diathermy forceps and cables that are configured into two separate types, namely bipolar and mono-polar. These diathermy forceps are normally used in surgical procedures to burn wounds that are prone to bleeding so preventing such bleeding during surgery. They are also employed to provide a final situation from which the wound can heal once the operation is concluded. Both techniques are used extensively in surgery throughout the world.

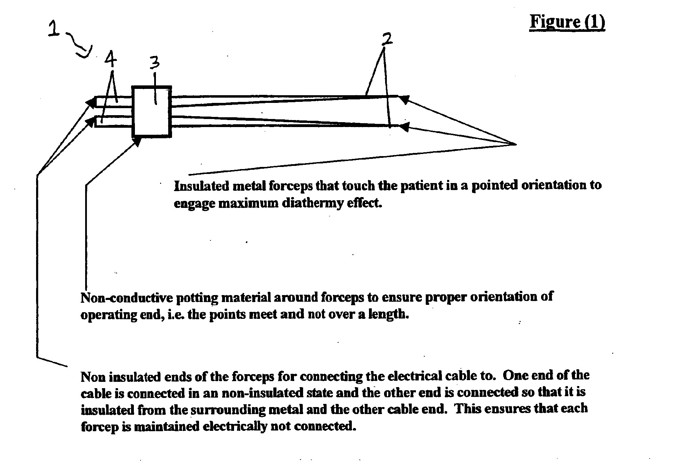

The current basic design of diathermy forceps 1 is presented schematically in Figure 1. Diathermy forceps can be seen to comprise insulated metal forceps 2, a nonconductive mount 3 and two non-insulated electrical cable connectors 4. It is the insulated metal forceps 2 that produce the required diathermy effect on the patient when they come into contact with the patient. The nonconductive mount 3 helps to ensure the proper orientation of the operating end. Either a mono-polar cable or a bipolar cable (not shown) is attached to the electrical cable connectors 4. These cables allow the required electrical current to be administered to the patient from an appropriate mono-polar or bipolar power generator (not shown) that is normally present in the theatre.

Presently a surgeon who wishes to use a mono-polar diathermy forceps requires to be given the appropriate metal forceps and electrical supply cable that has been manufactured for that purpose. Similarly the surgeon seeking to use bipolar diathermy forceps must be supplied with a completely different set of metal forceps and electrical supply cable. This has the obvious disadvantage that two separate sets of diathermy forceps must be present thus adding to the expense and congestion within a crowded operating theatre.

It is an object of the present invention to provide a connection means for diathermy forceps that is suitable for use with either a bipolar or a mono-polar power supply.

According to a first aspect of the present invention there is provide a diathermy forceps power supply connection means comprising an insulated connector characterised in that said insulated connector comprises a first power supply male, a first female connection port and a first forceps electrical supply means such that the connection means is suitable for use with either a bipolar or a mono-polar power supply.

Most preferably the power supply connection means further comprises a second insulated connector, wherein said second insulated connector comprises a second power supply male and a second forceps electrical supply cable.

Preferably the second insulated connector further comprises a second female connection port.

Most preferably a power supply male are suitable for connection with a female connection port and female connection ports associated with the bipolar and mono-polar power supply.

Preferably the forceps electrical supply means comprise an electrical cable.

According to a second aspect of the present invention there is provided a method of employing the diathermy forceps power supply connection means, suitable for connecting a set of diathermy forceps to a bipolar power supply, so as to enable the set of diathermy forceps to be connected to a mono-polar power supply characterised in that the method comprises the following steps:
(a) Plugging a power supply male of a first connection means into a female connection port of the mono-polar power supply; and
(b) Plugging a power supply male of a second connection means into a female connection port of the first connection means.

An embodiment of the present invention will now be described by way of example only with reference to the accompanying Figures, in which:
- Figure 1: presents a schematic illustration of standard diathermy forceps;
- Figure 2: presents a diathermy forceps power supply connection means in accordance with an aspect of the present invention; and
- Figure 3: presents the diathermy forceps power supply connection means of Figure 2 configured for use with:
(a) a bipolar power supply; and
(b) a mono-polar power supply.

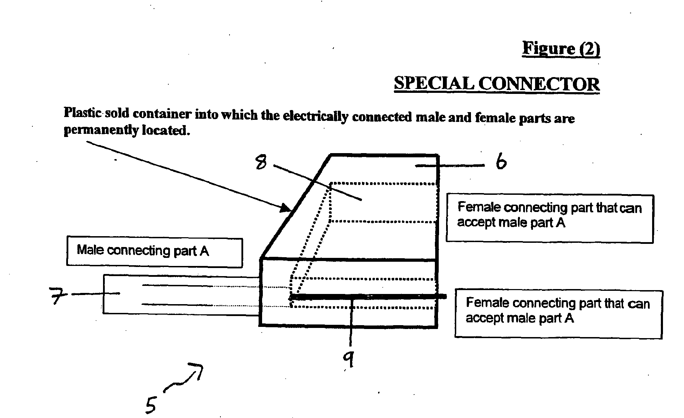

Figure 2 presents a schematic representation of a diathermy forceps power supply connection means 5. The diathermy forceps power supply connection means 5 can be seen to comprise an insulated connector 6, a power supply male 7, a female connection port 8 and a forceps electrical supply cable 9.

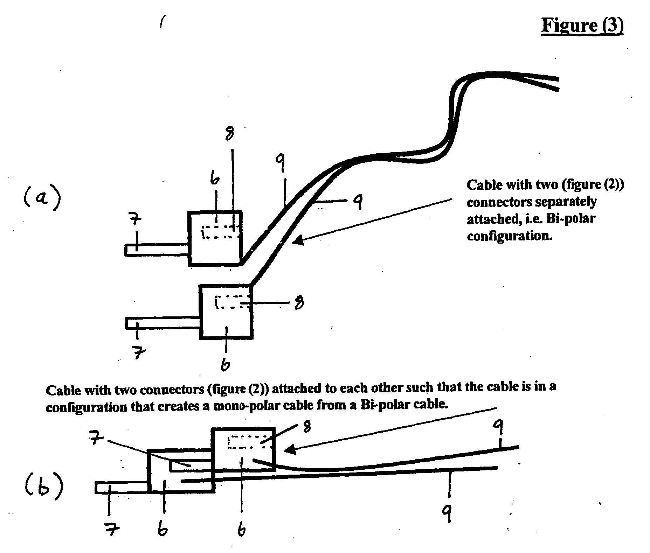

Two diathermy forceps power supply connection means 5 are employed to provide electrical power to the metal forceps (not shown). The configuration of the two diathermy forceps power supply connection means 5, for use with a bipolar power supply and a mono-polar power supply, are shown in Figure 3(a) and Figure 3(b), respectively.

When a surgeon requires bipolar diathermy forceps the diathermy forceps power supply connection means 5 are plugged, via the power supply males 7, directly into the bipolar power supply. The metal forceps then act as bipolar diathermy forceps as is well known in the art.

However, when the surgeon requires mono-polar diathermy forceps they are required to plug the first diathermy forceps power supply connection means 5 into the second via the second female connection port 8. Thereafter, the second diathermy forceps power supply connection means 5 is plugged via the second power supply male 7 directly into the mono-polar power supply. The same metal forceps, employing the same electrical supply cables, can then act as mono-polar diathermy forceps, as is well known in the art. By simply reversing the above process the metal forceps can be easily converted back so as to once again act as bipolar diathermy forceps.

It will be obvious to one skilled in the art that the above process of employing a mono-polar power supply could also be achieved by employing just one diathermy forceps power supply connection means 5 and a standard connection means. The standard connection means need only comprises an insulated connector, a power supply male and a forceps electrical supply cable. In this embodiment the power supply male of the standard connection means would be plugged into the female connection port 8 of diathermy forceps power supply connection means 5. Thereafter, the power supply male 7 of the of diathermy forceps power supply connection means 5 would be plugged directly into the mono-polar power supply.

The present invention has the advantage that it provides a diathermy forceps power supply connection means that is suitable for use with either bipolar or mono-polar power supplies.

A further advantage of the present inventions is that employment of the diathermy forceps power supply connection means allows a user to employ the same metal forceps and electrical supply cables with either the bipolar or mono-polar power supply. Once the bipolar forceps have been converted to mono-polar forceps they can be easily converted 'back by simply reversing the conversion steps.

The foregoing description of the invention has been presented for purposes of illustration and description and is not intended to be exhaustive or to limit the invention to the precise form disclosed. The described embodiments were chosen and described in order to best explain the principles of the invention and its practical application to thereby enable others skilled in the art to best utilise the invention in various embodiments and with various modifications as are suited to the particular use contemplated. Therefore, further modifications or improvements may be incorporated without departing from the scope of the invention herein intended.

## Claims

1. A diathermy forceps power supply connection means (5) comprising an insulated connector (6) **characterised in that** said insulated connector (6) comprises a first power supply male (7), a first female connection port (8) and a first forceps electrical supply means (9) such that the connection means (5) is suitable for use with either a bipolar or a mono-polar power supply.

2. A diathermy forceps power supply connection means (5) as claimed in Claim 1 wherein the connection means (5) further comprises a second insulated connector (6), wherein said second insulated connector (6) comprises a second power supply male (7) and a second forceps electrical supply means (9).

3. A diathermy forceps power supply connection means (5) as claimed in Claim 2 wherein the second insulated connector (6) further comprises a second female connection port (8).

4. A diathermy forceps power supply connection means (5) as claimed in any of the preceding claims wherein a power supply male (7) is suitable for connection with a female connection port (8) and female connection ports associated with the bipolar and mono-polar power supply.

5. A diathermy forceps power supply connection means (5) as claimed in any of the preceding claims wherein the forceps electrical supply means (9) comprises an electrical cable.

6. A method of employing a diathermy forceps power supply connection means (5), suitable for connecting a set of diathermy forceps (1) to a bipolar power supply, so as to enable the set of diathermy forceps (1) to be connected to a mono-polar power supply **characterised in that** the method comprises the following steps:
(a) Plugging a power supply male (7) of a first diathermy power supply connection means (5) into a female connection port (8) of the mono-polar power supply; and
(b) Plugging a power supply male (7) of a connection means into the female connection port (8) of the first diathermy power supply connection means (5).

7. A method of employing a diathermy forceps power supply connection means (5) as claimed in Claim 6 wherein the connection means comprises a second diathermy power supply connection means (5).
